# EUROPEAN PATENT SPECIFICATION

(11) **EP 3 750 576 B1**
(45) Date of publication and mention of the grant of the patent: **21.01.2026**
(21) Application number: 19179297.7
(22) Date of filing: 11.06.2019
(51) Int. Cl.: A61M 5/20, A61M 5/142, G09G 3/32, H04N 5/58

(54) **LED SENSOR FOR DRUG DELIVERY DEVICE**
LED-SENSOR FÜR ARZNEIMITTELABGABEVORRICHTUNG
CAPTEUR À DEL POUR DISPOSITIF D'ADMINISTRATION DE MÉDICAMENTS

(43) Date of publication of application: 16.12.2020
(73) Proprietor: Ypsomed AG, 3401 Burgdorf (CH)
(72) Inventor: Rufer, Thomas, 3072 Ostermundigen (CH)
(74) Representative: Meier Obertüfer, Jürg

(56) References cited:
- EP-A1- 2 162 167
- EP-A1- 3 476 417
- EP-B1- 2 162 167
- WO-A1-2016/126585
- WO-A1-2018/064784
- US-A1- 2008 224 027
- US-A1- 2018 090 290
- US-B2- 7 759 627

## Description

### FIELD OF THE INVENTION FIELD

The present invention relates to drug delivery systems for delivering, administering, injecting, infusing or dispensing liquids comprising a drug, medicament, or active ingredient. Preferred variants of the invention depart from a drug delivery device or an electronic module attachable to the drug delivery device with a LED used as a light source.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Exemplary drug delivery devices are known from EP 3 476 417 and EP 2 162 167.

In order to improve the ongoing therapy and to reduce the risk of a false administration modern drug delivery devices may comprise means for monitoring the injection process or/and the handling of the drug delivery device. The monitoring means may comprise an electronic circuit adapted to recognize, monitor and record injection or infusion events executed by means of the drug delivery device and to keep track of the doses already applied. The electronic circuit is further adapted to forward and/or display the recorded drug delivery data to a user. For example, an electronic drug delivery device or an electronic module attached to the drug delivery device and comprising such an electronic circuit may assist the user in real time during the injection process by providing step-by-step instruction on a display. Furthermore, the electronic circuit may give visual and/or acoustic feedback for particular events such as, for example, the termination of successful injection process. US 2008/224027 generally discloses a display device in which an ambient light photosensor that senses the brightness of ambient light is incorporated into a display panel, and a light source that illuminates the display panel is controlled according to an output of such ambient light photosensor.

These electronic circuits or monitoring means may be either embedded in a preferably reusable drug delivery device itself (e. g. in an electronic injection pen) or the electronic circuit may be in a separate electronic module releasably attachable to a preferably disposable drug delivery device. Such electronic modules are also named as "smart device", "smart module", or "add-on". The electronic module has to be mechanically coupled to the drug delivery device prior the injection or infusion process.

The data recorded or generated in the electronic circuit are usually forwarded to an external computing device such as a computer, a user mobile phone or to a server. In order to communicate from an external device to the electronic circuit in the drug delivery device or in the electronic module the electronic circuit is connected to the external computing device by a wireless communication link.

There are several wireless communication links known in the art such as a local WLAN or any other short or near range wireless communication technology such as Bluetooth, ZigBee, WiMAX or NFC. Besides that, there exits optical wireless communication technologies using light as medium to deliver signals. The technology is also known as Li-Fi (light fidelity) communication.

US 20180090290 A1 discloses a one way transmission of signals from an external device to in-vivo sensing capsules. The capsules comprise an electronic circuit including LEDs and a bi-directional gate to enable selective operation of the LEDs in a continuous light source mode, to illuminate the gastrointestinal tract and in a light sensing mode to receive a light signal indicative of a command to activate or deactivate devices in the capsule. In the light source mode the bi-directional gate connects a LED driver to the LEDs via a first path to transfer power to the LEDs. In the light sensing mode the bi-directional gate electrically disconnects the first path and creates a second path in reversed direction that electrically connects the LEDs to a light sensing circuit, so that the LEDs operate as a light sensors.

WO 2016126585 A1 discloses a visible light communication for secure transfer of data within an operating room. A visible light communication (VLC) enabled mobile device comprises a LED for transmitting the data and a photodiode for receiving data. The data are transferred by targeting the light transmitting portion of the mobile device in the line of sight of a light detecting sensor of a medical VLC enabled device e.g. a robotic surgical system. The data include patient information with their diagnosis, medical history, surgical procedure, pre-operative images or plans.

### DESCRIPTION OF THE INVENTION

It is an objective of the invention to provide for communication in a space and component-saving design in a drug delivery system. This objective is achieved by a drug delivery device, an electronic module releasably attachable to a drug delivery device and a method according to the independent claims. Preferred embodiments are evident from the dependent patent claims.

According to the invention an electronic module releasably attachable to a drug delivery device and being adapted for monitoring of a drug delivery event executed by means of the drug delivery device and/or optionally for reading drug data of a drug comprised in a container of the device, or device data of the delivery device, comprises a LED component with at least two connector pins and a controller adapted for alternatingly operating the LED component in a first and in a second state. In each second state, the controller monitors a voltage, preferably a voltage decay, across the LED component between the at least two connector pins, to determine a sequence of discrete changes in light intensity absorbed by the LED component, and to derive a device input signal, preferably a binary device input signal, from the sequence. In the first state the LED component is operated to emit light pulses of an output signal or representing an output signal. Preferably, the LED component is operated to emit light pulses based on the device input signal.

The invention relates further to a drug delivery device with a container for holding a liquid drug and a cannula or needle for delivery of the drug, said drug delivery device comprising a LED component with at least two connector pins and a controller adapted for alternatingly operating the LED component in a first and in a second state. In each second state, the controller monitors a voltage, preferably a voltage decay, across the LED component between the at least two connector pins, to determine a sequence of discrete changes in light intensity absorbed by the LED component, and to derive a device input signal, preferably a binary device input signals, from the sequence. In the first state the LED component is operated to emit light pulses of an output signal. Preferably, the LED component is operated to emit light pulses based on the device input signal.

According to the invention, a LED (Light Emitting Diode) in a medical device is used for both signal transmission and signal reception in an optical communication link to the device. A dual use of the LED allows saving components and space, which results in lighter and smaller and therefore more user-friendly devices. The medical device in particular is an electronic delivery device or an electronic module for a delivery device.

The transmission of signals based on light does not influence electromagnetic fields. This is advantageous in particular for devices sensitive to electromagnetic fields because the light communication does not affect the sensitive devices. Furthermore, the use of a LED component allows a power efficient operation because the LED component uses only minimal energy compared to conventional sender and receiver of a wireless network.

Since the LED is capable of emitting light in a first state as well as sensing a light intensity in a second state, a communication link between the drug delivery device or the electronic module and an external device adapted to emit a light signal and to detect a light signal can be established.

The LED component comprises at least two connector pins serving for electrically contacting the light emitting diode to conductive paths of an LED support onto which the LED is mounted, such as a printed circuit board (PCB), a flexible or semi-flexible PCB. One pin may contact the LED to a positive pole and the other of the two pins may contact the LED to a negative or to the ground pole.

The controller is adapted to operate the LED component in first state, in which the LED component emits light. Preferably, the controller is adapted for operating the LED component in a pulse modulation mode for intermittently generating light pulses separated by idle periods. Several consecutive pulses may form a light output signal. The output signal can be detected and read by an external receiver. The external receiver may comprise, for example, a LED sensor, a photodiode, a photomultiplier or any sensor adapted to detect light intensity and to recognize the light pulses. Preferably, the LED component is operated in the first state with a first bias voltage. In the first state, the LED may operate in a continuous, blinking, or mixed mode, at maximum or reduced power, immediately signaling a device or delivery status (ok, ready, warning) to a person observing the LED. Alternatively, or superposed to the human-perceivable quasi-static status signal, drug data, device data, delivery event data with enhanced information may be transmitted via the LED, by emitting short light pulses of variable duration and separated by idle periods.

In a preferred embodiment the drug delivery event data, device data or drug data are stored in a storage unit in the drug delivery device or in the electronic module. These data are preferably prepared by a processor or by the controller and transmitted via LED component to the external receiver.

The controller is further adapted for operating the LED component in a second state different from the first state. In the second state the same LED component does not or does emit only very little light compared with the first state. Preferably, the LED component is operated in the second state with no voltage being applied, preceded by applying a second bias voltage reversed to the first bias voltage. For operating the LED component with the reversed bias voltage the voltage may be simply reversed when switching from the first state to the second state or from the second state to the first state.

In a preferred embodiment in a first period in the second state the LED component is operated with a reversed bias voltage and in a second period in the second state the LED component is operated with no applied voltage.

In the second state the LED component operates as light sensor in the manner of a photodiode. Following charging of a residual capacity of the LED component by the reverse bias voltage, the latter is stopped, and a decay of the pin voltage (change of voltage across the LED) is observed. Therefore, the controller is adapted to measure a voltage between the two connector pins of the LED component in the second state. Preferably, the voltage across the LED component is detected by a high-impedance measurement and the voltage is measured in several cycles or at several times to determine a chronological sequence of a voltage levels or a voltage decay.

The decay is depending on incoming photons on the LED component. That means, the behavior of the decay gives conclusions of the light intensity of the light absorbed by the LED component in the second state. The light intensity may not change. Even a constant light intensity causes a decay, which can be detected. Furthermore, a change in light intensity in between successive measurements can be detected.

If the determined light intensity changes several times a specific or predetermined sequence of changes may be detected. Subsequently, the controller is adapted to derive a device signal from the sequences of discrete changes of the light intensity based on a predefined pattern, list or database. Such a signal comprises usually several light pulse, which last, for example, several microseconds or milliseconds.

Furthermore, the controller may be calibrated in order to be able to determine an absolute light intensity. That means the controller is adapted to determine a specific value of the light intensity based on a specific measured decay or rate of change of the voltage.

The controller is adapted to determine a sequence of changes in the absorbed light intensity. That means the change is determined if successive light intensities differs in a predefined minimum from each other. In other words, the difference of the light intensity exceeds a predefined threshold between a first light intensity determined based on a first measuring point or measuring instant and a second light intensity determined based on a second measuring point or measuring instant.

The LED component may be operated by a LED driver. The LED driver can be integrated in the controller or it can be a separate component but controlled by the controller. The measuring of the voltage may be carried out by the controller itself or by a measurement device controlled by the controller. Hence, the operating of the LED component and the measuring of the voltage across the LED component may be realised by one device or by a plurality of separate devices.

The absorbed light intensity may originate from an external device in form of a user mobile device or an external computer or from a light source, for example, from a lamp being present in close vicinity of the LED component, or may simply represent ambient light. Preferably, the controller or another component is adapted to read out data from the recognized input signal, e.g. the light input signal is processed to derive at least a binary value or single bit of binary optical data directed to the drug delivery device or electronic module.

Preferably, the controller is adapted for operating alternatingly the LED component in the first state and in the second state. The term "alternatingly" means the controller switches the LED component from the first state direct or indirect to the second and back direct or indirect to the first state and so on. The term "indirect" means the controller may switch the LED component to a third or any other state, which differs from the first and the second state. Furthermore, the controller may not switch the LED component in each duty cycle, e.g. the LED component may be switch several cycles between the first and the third state and then in one cycle between the first and the second state before again switching between the first and third state. In another embodiment the controller may switch the LED component from the first state to a third state and then to the second state and back to the first state and so on. In a preferred embodiment the controller is adapted in the third state to operate the LED component with a zero voltage or ground voltage.

Thus, the controller or a LED driver, which is controlled by the controller switches the LED component alternatingly between the first and the second state. That means LED component alternatingly generates an output signal by emitting light pulses (first state) and determines an input signal by measuring the voltage across the LED component (second state).

The time periods the LED component is operated in the first or second state can be equal or not. In a preferred embodiment the time period of the first state (generating output signal) is shorter than the time period of the second state (determining input signal). For example, the time period the LED component is operated in the first state may last several microseconds. The time period the LED component is operated in the second state may last, for example, several milliseconds.

The drug delivery device may be an infusion system or an injection system. The injection system may be an electronic injection device adapted to provide drug delivery event data (injection or infusion data), device data or drug data. The electronic injection device may be, for example, an electronic injection pen or it may be a patch injector applicable onto the skin of the user for the duration of the injection. Alternatively, the injection system may not comprise electronic components for providing drug delivery data. Instead, the injection system may comprise an electronic module releasably attachable to an injection device and adapted to provide the drug delivery data.

The infusion system may be, for example, a conventional medical drug pump such as an insulin pump with tubing or it may be a drug patch pump without tubing that is attachable directly onto the skin of the user. The infusion system may be adapted to recognize, monitor and record infusion processes executed by means of the infusion system and to provide drug delivery event data.

As descripted above the drug delivery device itself or an electronic module attachable to a drug delivery device may be adapted to recognize, monitor and record injection or infusion events executed by means of the injection or infusion device and to keep track of the doses already applied. For this purpose, drug delivery event data may be generated in the delivery device or in the electronic module. Drug delivery event data may be, for example, an administered dose of the drug or the time and date of an administration. The drug delivery device or the electronic module may give visual and/or acoustic feedback for particular events such as, for example, the termination of successful injection or infusion process.

Instead or additionally, the delivery device or the electronic module may be adapted for optionally reading drug data of a drug comprised in a container of the device, or device data of the delivery device. Such drug data may be, for example, the type of the drug, the expiration data or a lot number of the drug. The device data may comprise, for example, the type of the drug delivery device, a lot number of the device or a fabrication data of the device. The drug delivery device or the electronic module may be further adapted to forward and/or display the recorded drug delivery event data, the device data or the drug data to an external device.

The term "injection system" or "injection device" is to be understood as a delivery device that is removed from the injection site after each medication event or drug delivery process. Contrary, the term "infusion system" refers to a drug delivery device with a cannula or needle that remains in the skin of the patient for a prolonged period of time, for example several hours.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

In the present description, the term "distal" refers to a direction pointing to the injection site or to end of the drug delivery device where an injection needle or cannula is located. Correspondingly, the term "proximal" refers to a direction pointing away from the injection site or to a direction to the end of the drug delivery device located furthest away from the injection site.

The term "longitudinal", with or without "axis", refers to a direction or an axis through the device or components thereof in the direction of the longest extension of the device or the component. The terms "radial" refers to a direction through the device or components thereof in a direction generally perpendicular to the longitudinal direction.

In a preferred embodiment the electronic module or the drug delivery device comprises a sensor unit for detecting a drug delivery event and/or for detecting device data or drug data of a drug being present inside the drug delivery device, wherein the sensor unit is adapted for storing generated drug delivery event data, the drug data or the device data.

For example, the sensor unit is capable of recording and saving an injection or infusion events together with a time stamp. Such events may be detected by analysing a motion sensor signal of the sensor unit. That means a specific pattern of motion may be indicative for an injection executed by the drug delivery device. In order to detect device data or drug data the sensor unit may be, for example, adapted for reading an identification code such as a RFID of the delivery device or of the drug inside the delivery device.

The sensor unit may further comprise a long or short term storage in order to store the drug delivery event data, the device data or the drug data. In this case, the controller may be adapted to read the data from the storage and subsequently generate the output signal based on the read data.

Preferably, the output signal representing the delivery event data, the device data or the drug data is generated based on the device input signal. That means the output signal is a response to request send by an external receiver. Hence, an optical communication is established. Preferably, the optical communication is the exclusive communication link between the drug delivery device or the electronic module and the external device. Furthermore, the optical communication may even supplant any other wireless communication link such as Bluetooth or the like for the purpose of output data transmission. This allows to save battery capacity because the LED component consumes little electrical energy compared to a conventional data receiver or data transmitter component.

In a preferred embodiment the drug delivery event data comprise at least one of the following information; an amount of the last administered dose, indication of time of the last administered dose, holding time, dialed dose, amount of the next dose to be administered, indication of time of the next dose to be administered or total amount of administered dose.

These information may give evidence of the injection or infusion status and may help to analyse, monitor or /and improve the administration process.

Preferably the drug data comprise at least one of the following information; drug type, expiration of the drug, or validity of the drug. In case the drug delivery device or the electronic module attached to the drug delivery device comprises a temperature sensor adapted for measuring the temperature of the drug data may comprise actual and/or stored temperature information.

Preferably, the controller is adapted to measure a rate of change or a decay of the voltage between the two connector pins of the LED component. That means the rate of change is computed or derivated immediately or simultaneously during the measuring of the voltage level. Knowing the rate of change of the voltage or the decay allows to determine an absorbed light intensity in an efficient way. The measuring is carried out by a voltmeter, which may be integrated in the circuit or in the controller or it may be a separate device. Alternatively, the voltage may be measured in single measuring steps and the measured voltage levels are compared at a later stage.

In a preferred embodiment the device input signal represents configuration data for configuring the electronic module or the drug delivery device and the controller is adapted to read the configuration data from the device input signal. That means the controller is adapted to extract the configuration data form the device input signal. Subsequently, the electronic module or the drug delivery device can be configured according to the configuration data by the controller or any computing unit. The configuration data may be sent via light signal from an external computing device such as a user mobile device or a computer having direct visual contact to the electronic module or the drug delivery device, respectively.

The external computing device may send the configuration data to wirelessly configure the drug delivery device, e.g. to carry out a priming, to set a predefined dose or to adapt the drug delivery settings to a specific drug inside the drug delivery device.

Instead or additionally, the device input signal preferably represents pairing data for pairing the electronic module or the drug delivery device with a computing device and wherein the controller is adapted to read the pairing data.

The pairing data may be sent from an external computing device, for example, from a user mobile device or a computer. The external computing device sends preferably a pairing request in order to pair the computing device with the drug delivery device or with electronic module, respectively, prior to the transferring of medical data. That means the output signal representing the drug delivery data or the data relating to the drug is send after the pairing procedure.

In order to start the pairing a pairing response is generated and sent after the controller has extracted the pairing data from the input signal. For that purpose, the controller operates the LED component in the first state to emitting light pulses to generate a light signal representing the pairing response. The pairing allows to establish a secure communication link between the drug delivery device or the electronic module and the external computing device.

The pairing via light signal is advantage because the pairing can be carried out even if other communication links such as NFC or Bluetooth do not work or cannot be applied.

Preferably, the optical communication is the exclusive communication link between the drug delivery device or the electronic module and the external device used for the purpose of pairing, in particular replacing a touch-to-pair and/or NFC based pairing mechanism between the electronic module or the drug delivery device and the external device. Furthermore, the optical communication may even supplant any other wireless communication link such as Bluetooth or the like for the purpose of output data transmission.

Preferably, the output signal represents an identification code of the electronic module or the drug delivery device. The identification code may be any number, key or figure unambiguously identifying the electronic module or the drug delivery device. The identification code is preferably transmitted in a separate signal prior to any other data transmission.

Preferably, the controller recognizes and reads an input signal from a computing device prior to the generation of an output signal representing the identification code. The input signal comprises a pairing request or pairing data in order to start the pairing procedure.

The pairing may comprise the exchange of pairing keys or access rights such that a secure communication link may be established between the drug delivery device or the electronic module attached to the drug delivery device and the external computing device. The external computing device may be, for example, a user mobile phone, a tablet computer, a notebook computer or any other portable or wearable computing device.

Preferably, the controller is adapted to start processing data if the device input signal is derived from the sequence of changes in the absorbed light intensity.

The controller may be in a power saving modus with reduced processing activity but with ongoing controlling and processing of the measuring of the voltage. When a predefined sequences of discrete changes in the absorbed light intensity is detected the controller switches from the power safe mode to normal operation mode and starts to process data, for example, to extract data from the derived input signal. Furthermore, if a predefined sequence is detected the processor may activate or wake up connected electronic components from a power save mode to normal operation mode.

Preferably, the LED is a RGB-LED. This LED offers flexibility of emitting different colors or even mixing different RGB colors. Alternatively, a one color LED may be used.

The invention relates further to a method for a drug delivery device with a container for holding a liquid drug and a cannula or needle, the method comprises the steps of:
a. Operating, by a controller of the drug delivery device, a LED component in a second state;
b. Monitoring, by the controller, in the second state a voltage decay across the LED component between two connector pins of the LED component;
c. Deriving, by the controller and based on the monitoring, an ambient light parameter value depending on the input light intensity absorbed while monitoring;
   and preferably deriving, by the controller, a change in the parameter value between successive monitorings;
d. Operating, by the controller, the LED component in a first state and emitting light with an intensity based on the latest ambient light parameter value or the change of the parameter value, wherein the intensity is human eye perceptible.

A present ambient light may be detected if there is no decay or a very low rate of change in the derived absorbed light intensity. That means the light intensity in the surrounding of the LED component is more or less constant and there is no pulsing or flashing light source. In this embodiment the generated output is a constant light intensity emitted by the LED component. The emitted constant light intensity is adjusted to the ambient light intensity, the term constant referring to a perception by the human eye and not excluding a modulation of the LED at high frequency. For example, the light intensity of a display in the drug delivery device or in the electronic module is adjusted to the ambient light intensity such that elements shown in the display are easy to read.

According to the invention the above described method can also be carried out with an electronic module releasably attachable to a drug delivery device and being adapted for monitoring of a drug delivery event executed by means of the drug delivery device.

A further method is disclosed which is not related and not covered by the present invention. This method relates to a transmission of a signal by a LED component of a drug delivery device with a container for holding a liquid drug and a cannula or needle for delivery of the drug or of an electronic module releasably attachable to the drug delivery device. The method comprising the steps of:
a. Operating, by a controller of the drug delivery device or the electronic module, a LED component in a second state;
b. Monitoring, by the controller, in the second state a voltage across the LED component between two connector pins of the LED component;
c. Determining, by the controller and based on the monitoring, a sequence of discrete changes in light intensity absorbed by the LED component;
d. Deriving, by the controller, a device input signal from the sequence;
e. Operating, by the controller, the LED component in a first state;
f. Emitting, by the LED component, in the first state light pulses representing an output signal representing drug delivery event data, delivery device data or drug data based on the device input signal.

In a preferred embodiment not according to the present invention before step a) the method comprises the steps of
i. Recognizing, by a sensor unit of the drug delivery device or of the electronic module, a drug delivery event executed by the drug delivery device;
ii. Generating, by the sensor unit, the drug delivery event data based on the recognized drug delivery event;
iii. Storing, by the controller, the generated data in a storage unit of the drug delivery device or of the electronic module.

The drug delivery event may be recognized by detecting a movement or a specific setting of the drug delivery device. For example, a movement of the drug delivery device may be sensed by a motion sensor and the controller or a processor of the drug delivery device or the electronic module may analyse the sensed motion signal in order to determine whether a drug delivery has been occurred. Subsequently, the data generated during the analysing of the signal or generating after the analysis are stored in the storage unit by the controller.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an electronic module realisably attached to an auto-injector;
- Fig. 2: depicts a side view of an electronic injection pen with an LED;
- Fig. 3: schematically depicts an electronic circuit of a LED component operated in forward bias;
- Fig. 4: depicts the circuit of figure 3, wherein the LED component is operated in reversed bias and
- Fig. 5: depicts the circuit of figure 4 during monitoring.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

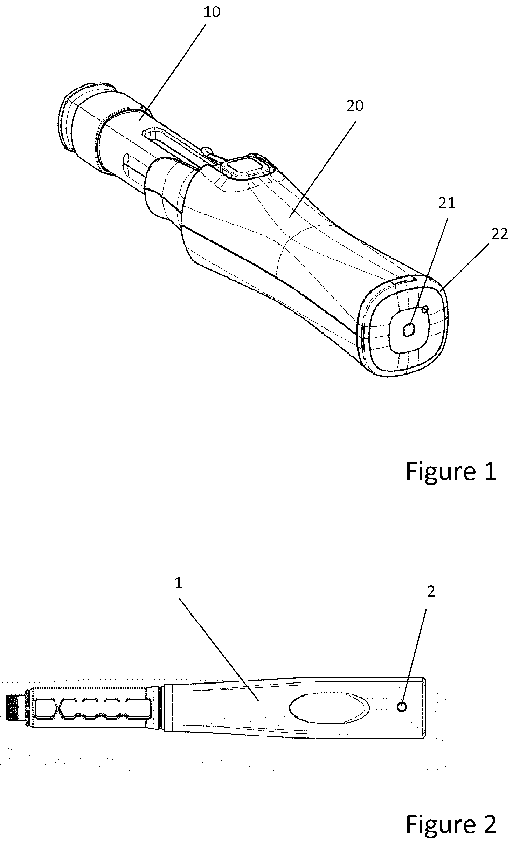

Figure 2 depicts an electronic module 20 according to the invention that is releasably attached to a device housing of a drug delivery device. In this embodiment the drug delivery device is disposable auto-injector 10, which itself is not adapted for recording or/and storing data from the injection process. Instead, the electronic module 20 can record, store and process pairing, configuration and drug delivery data as described below.

The electronic module 20 includes a spot-shaped RGB-LED 21 being located on a rear or proximal end surface of the electronic module 20, and a ring-shaped RGB-LED element 22 being located around the proximal edge of the electronic module 20.

Furthermore, the electronic module 20 comprises a communication module connected to the LED 21 and 22 and a controller, a storage unit, a power supply and a sensor unit inside the housing of the electronic module 20. The sensor unit is adapted to monitor and record an injection process executed by the injector 10.

The LED 21 and 22 of the communication module can be used for generating output light signals as well as for detecting input light signals. The electronic module 20 can establish a communication link via LED with an external mobile device such as a smart phone, a tablet computer or a notebook computer. The communication link based on light via LED can be used to transmit data from the electronic module to the external mobile device or from the external mobile device to the electronic module.

The data may comprise a paring request sent by the mobile device to the electronic module. The latter may start the pairing procedure by sending a pairing key back via light signal to the mobile device.

Furthermore, the LED 21 and/or the LED 22 of the electronic module can be used as light sensor in order to receive configuration data for the injection pen. Such data may comprise settings for the pen to adjust dosing steps to the drug inside the pen or to any setting for the administration of the drug such as a command for carry out a priming, a command for setting a dose, for starting administration of a dose or for locking or unlocking the injection pen. In contrast, the LED as light emitter is used to generate an output signal in order to transmit data from the electronic module, for example, recorded or stored in the storage unit of the electronic module drug delivery data such as injection status, time and data of an administration, the amount of the last administration, an identification code from the drug inside the injector 10 or/and the current settings of the injector 10.

However, the controller in the communication module may not only sense an input signal representing data but also sense ambient light being present in the close vicinity of the LED.

The electronic module may comprise a power safe mode in order to preserve the restricted electrical power source in the electronic module. When an incoming light signal is detected the controller switches from the power safe mode to normal operation mode and starts to extract data from the input signal.

Figure 2 depicts a further embodiment of the invention, namely a drug delivery device in the form of an electronic injection pen 1. The pen 1 comprises an injection needle or cannula at the distal end (not shown) and an electronic unit at the proximal end inside a housing of the pen. The delivery device may comprise a drive for automatically driving a piston rod in the pen to administer the drug. The injection pen 1 can record, store and process pairing, configuration and drug delivery data as described in relation with the electronic module 20 above.

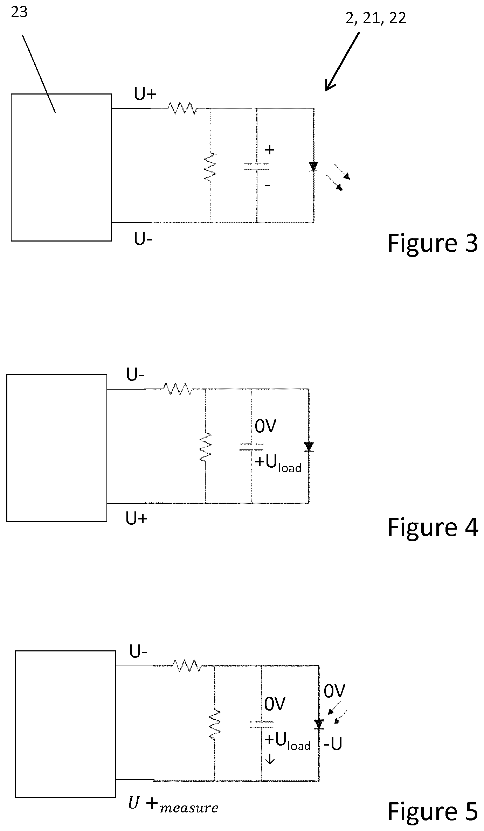

In the following and referring to figure 3 to 5 the function of the optical communication by means of the LED 2 of the injection pen 1 or of the LED 21 or 22 of the electronic module 20 is described in detail.

The electronic injection pen 1 or the electronic module 20 attached to a injector comprises a controller (not shown) in an integrated circuit, in particular a microcontroller, adapted for operating the LED 2, 21, 22. The controller is electronically connected to the LED 2, 21, 22.

Figure 3 shows an electric circuit diagram of a LED component in detail. In reality, the LED has not only a behavior of a diode but comprises additionally also a capacitor like and a resistor like behavior. These effects are also called parasitic capacitance and parasitic resistance. In order to take these effects into account the LED circuit in figure 3 is drawn not only with a diode but also with a capacitor component and resistor components. For the sake of simplicity, in the following these parasitic components are named as capacitor and resistor, respectively, despite the fact that the LED component does not comprise physically a capacitor and a resistor component.

The circuit comprises a first branch or diode anode branch (upper branch in figure 3) connecting a first connector pin of the light emitting diode to a first pole. The circuit comprises further a second branch or diode cathode branch (lower branch in figure 3) connecting a second connector pin of the diode to a second pole. The circuit comprises a first resistor in the first branch representing the wire resistance. Furthermore, the circuit comprises a second resistor parallel to the light emitting diode and further a capacitor parallel to the diode.

In order to emit light the controller operates the LED component in a first state, in that a forward bias voltage is applied. That means a voltage source is connected to the first and second branch and applies a positive voltage to the first branch, as shown in figure 3. The voltage being present across the capacitor is depicted with a "plus" and a "minus" sign in figure 3.

The LED can then be operated by the controller, for example, in a pulse modulation to adjust the light to a specific intensity or to generate relatively short light pulses. The controller is adapted to operate the LED to generate a signal representative for pairing data, configuration data, drug delivery data or data relating to the drug in the device to be transmitted via light pulses by the LED component. Thus, the controller converts the data into a light signal.

Figure 4 shows the same circuit of the LED component but in the depicted situation the LED is operated in a second state. In contrast to the first state a negative voltage is applied the first branch and a positive voltage is applied to the second branch such that the LED is biased in reverse direction. A positive voltage is present at the second branch of the capacitor, marked as "+U_{load}" in figure 4. The diode is reverse biased and blocks current. The reversed-biased capacitor is charged at this stage.

Figure 5 shows the circuit of figure 4 but with light reaching the diode. The incoming photons reaching the diode cause the diode to work like a voltage source with very little power. The polarity of this light source is depicted in figure 5. There is a zero voltage at the first branch side (anode side) of the diode and a negative voltage on the second side (cathode side) of the diode. In other words, the diode works like a negative voltage source. Since the charged capacitor is reverse biased the capacitor is discharged and the voltage "+U_{load}" across the capacitor drops. The voltage across the capacitor can be measured with high-impedance measurement, preferably carried out cyclically.

If light reaches the diode with a high intensity this causes the capacitor to discharge faster than with light with a low intensity. If the discharging of the capacitor is measured cyclically the discharging time can be determined. Depending on this discharging time (decay or rate of change) the intensity of the light can be computed.

If the determined light intensity changes several times a predetermined sequence of changes may be detected. Subsequently, the controller is adapted to derive a signal from the sequences of changes of the light intensity based on a predefined pattern. The signal in turn represents data from the external device.

A duty cycle for emitting light and detecting a light intensity can be thus summarized in the following steps:
a. Operating the LED component with a forward bias voltage (first state), for emitting light, figure 3;
b. Operating the LED component with a revers bias voltage (second state), for charging the capacitor of the LED component, figure 4;
c. Measuring the discharging time of the capacitor (second state) for determining the intensity of light at the diode.

After step c. the LED component can again be used for emitting light and thus starting again with step a. After each measuring cycle the LED can be operated in the first state such that the LED emits light and can be used to illuminate the environment or to emit light signals based on light pulses.

The time period for charging the capacitor in the second state lasts several microseconds, in particular 1 to 10 microsecond. The time period for measuring the discharging time of the capacitor lasts several milliseconds, in particular up to 5 milliseconds.

Preferably, the LED component is operated in the first state in the pulse modulation modus generating a large number of light pulses per second of different pulse width to transmit a signal representing drug delivery data. The pulses may have, for example, a pulse width of 10 milliseconds. The idle time between two light pulses in a transmission can be used to execute the capacitor charging and the discharging time measurement of the capacitor and thus to detect and read an incoming light signal intensity. Data transmission and data reception may thus be handled in an interleaved mode. Alternatively, occasional determination of ambient light intensity may be postponed to the end of data transmission, or be inserted in a continuous illumination state of the LED during operation of the device.

### LIST OF DESIGNATIONS

- 1: Electronic injection pen
- 2: LED including junction capacitance and junction resistance
- 10: Auto-Injector
- 20: Electronic module
- 21: LED including junction capacitance and junction resistance
- 22: ring-shaped LED element
- 23: Controller

## Claims

1. An electronic module (20) releasably attachable to a drug delivery device (10) and being adapted for monitoring of a drug delivery event executed by means of the drug delivery device (10), said electronic module (20) comprising
a LED component (21, 22) with at least two connector pins,
**characterised in that**
the electronic module (20) further comprises a controller (23) adapted for alternatingly operating the LED component (21, 22) in a first and in a second state,
wherein in the second state, the controller monitors a voltage across the LED component (21, 22) between the at least two connector pins, to determine a sequence of discrete changes in light intensity detected by the LED component (21, 22), and to derive a device input signal from the sequence and
wherein in the first state the LED component (21, 22) is operated by the controller (23) to emit light pulses representing an output signal.

2. A module (20) according to claim 1, **characterised by** a sensor unit for detecting a drug delivery event and/or for detecting device data or drug data of a drug being present inside the drug delivery device (10), wherein the sensor unit is adapted for storing generated drug delivery event data, the drug data or the device data.

3. A module (20) according to claim 2, **characterised in that** the detecting of the drug delivery event comprises the detecting of at least one of the following; an amount of the last administered dose, indication of time of the last administered dose, amount of the next dose to be administered, indication of time of the next dose to be administered or total amount of administered dose.

4. A module (20) according to claim 2 or 3. **characterised in that** the drug data comprise at least one of the following information; drug type, expiration of the drug or validity of the drug.

5. A module (20) according to any of claims 1 to 4, **characterised in that** the controller is adapted to measure a rate of change of the voltage across the LED component (21, 22).

6. A module (20) according to any of claims 1 to 5, **characterised in that** the device input signal represents configuration data for configuring the electronic module (20) and wherein the controller is adapted to read the configuration data from the device input signal.

7. A module (20) according to any of claims 1 to 5, **characterised in that** the device input signal represents pairing data for pairing the electronic module (20) with a computing device and wherein the controller is adapted to read the pairing data.

8. A module according to claim 7, **characterised in that** the output signal represents an identification code of the electronic module (20) or the drug delivery device (10).

9. A module (20) according to any of claims 1 to 8, **characterised in that** the controller is adapted to start processing data if the device input signal is derived from the sequence.

10. A module (20) according to any of claims 1 to 9, **characterised in that** the LED component (21, 22) is a RGB-LED.

11. A drug delivery device (1) with a container for holding a liquid drug and a cannula or needle for delivery of the drug, said drug delivery device (1) comprising:
a LED component (2) with at least two connector pins,
**characterised in that**
the drug delivery device (1) further comprises a controller (23) adapted for alternatingly operating the LED component (2) in a first and in a second state,
wherein in the second state, the controller monitors a voltage across the LED component (2) between the at least two connector pins, to determine a sequence of discrete changes in light intensity detected by the LED component (2), and to derive a device input signal from the sequence and
wherein in the first state the LED component (2) is operated by the controller (23) to emit light pulses representing an output signal.

12. The drug delivery device of claim 11, **characterised in that** the device input signal represents configuration data for configuring the drug delivery device and wherein the controller is adapted to read the configuration data from the device input signal.

13. The drug delivery device of claim 12, **characterised in that** the controller is configured to switch from a power safe mode to a normal operation mode and to start processing data when a predefined sequences of discrete changes in the absorbed light intensity is detected.

14. A method for adjusting an emitted light intensity of a display in a drug delivery device (1) comprising the steps of:
a. Operating, by a controller (23) of the drug delivery device (1), a LED component (2) in a second state;
b. Monitoring, by the controller (23), in the second state a voltage decay across the LED component (2) between two connector pins of the LED component (2);
c. Deriving, by the controller (23) and based on the monitoring, an ambient light parameter value depending on the input light intensity detected by the LED component (2);
d. Operating, by the controller (23), the LED component (2) in a first state and emitting light with an intensity based on the latest ambient light parameter value.

15. A method for adjusting an emitted light intensity of a display in an electronic module (20) releasably attachable to a drug delivery device (10) and being adapted for monitoring of a drug delivery event executed by means of the drug delivery device (10), the method comprising the steps of:
a. Operating, by a controller (23) of the electronic module (20), a LED component (21, 22) in a second state;
b. Monitoring, by the controller (23), in the second state a voltage decay across the LED component (21, 22) between two connector pins of the LED component (21, 22);
c. Deriving, by the controller (23) and based on the monitoring, an ambient light parameter value depending on the input light intensity absorbed by the LED component (21, 22);
d. Operating, by the controller (23), the LED component (21, 22) in a first state and emitting light with an intensity based on the latest ambient light parameter value.

## Patentansprüche

1. Elektronisches Modul (20), das an einer Medikamentenabgabevorrichtung (10) lösbar anbringbar ist und zum Überwachen eines Medikamentenabgabeereignisses, das mittels der Medikamentenabgabevorrichtung (10) ausgeführt wird, geeignet ist, das elektronische Modul (20) umfassend
eine LED-Komponente (21, 22) mit mindestens zwei Anschlussstiften,
**dadurch gekennzeichnet, dass**
das elektronische Modul (20) ferner eine Steuereinrichtung (23), die zum wechselnden Betreiben der LED-Komponente (21, 22) in einem ersten und in einem zweiten Zustand geeignet ist, umfasst,
wobei in dem zweiten Zustand, die Steuereinrichtung eine Spannung über die LED-Komponente (21, 22) hinweg zwischen den mindestens zwei Anschlussstiften überwacht, um eine Sequenz diskreter Änderungen in Lichtintensität, die durch die LED-Komponente (21, 22) detektiert werden, zu bestimmen und um ein Vorrichtungseingangssignal aus der Sequenz abzuleiten, und
wobei in dem ersten Zustand die LED-Komponente (21, 22) durch die Steuereinrichtung (23) betrieben wird, um Lichtimpulse, die ein Ausgangssignal darstellen, zu emittieren.

2. Modul (20) nach Anspruch 1, **gekennzeichnet durch** eine Sensoreinheit zum Detektieren eines Medikamentenabgabeereignisses und/oder zum Detektieren von Vorrichtungsdaten oder Medikamentendaten eines Medikaments, das in der Medikamentenabgabevorrichtung (10) vorhanden ist, wobei die Sensoreinheit zum Speichern erzeugter Medikamentenabgabeereignisdaten, der Medikamentendaten oder der Vorrichtungsdaten geeignet ist.

3. Modul (20) nach Anspruch 2, **dadurch gekennzeichnet, dass** das Detektieren des Medikamentenabgabeereignisses das Detektieren mindestens einer von den Folgenden umfasst; einer Menge der zuletzt verabreichten Dosis, einer Zeitangabe der zuletzt verabreichten Dosis, der Menge der nächsten zu verabreichenden Dosis, der Zeitangabe der nächsten zu verabreichenden Dosis oder einer Gesamtmenge verabreichter Dosis.

4. Modul (20) nach Anspruch 2 oder 3, **dadurch gekennzeichnet, dass** die Medikamentendaten mindestens eine der folgenden Informationen umfassen; Medikamentenart, Ablauf des Medikaments oder Gültigkeit des Medikaments.

5. Modul (20) nach einem der Ansprüche 1 bis 4,
**dadurch gekennzeichnet, dass** die Steuereinrichtung geeignet ist, um eine Änderungsrate der Spannung über die LED-Komponente (21, 22) hinweg zu messen.

6. Modul (20) nach einem der Ansprüche 1 bis 5,
**dadurch gekennzeichnet, dass** das Vorrichtungseingangssignal Konfigurationsdaten zum Konfigurieren des elektronischen Moduls (20) darstellt, und wobei die Steuereinrichtung geeignet ist, um die Konfigurationsdaten aus dem Vorrichtungseingangssignal zu lesen.

7. Modul (20) nach einem der Ansprüche 1 bis 5,
**dadurch gekennzeichnet, dass** das Vorrichtungseingangssignal Kopplungsdaten zum Koppeln des elektronischen Moduls (20) mit einer Rechenvorrichtung darstellt, und wobei die Steuereinrichtung geeignet ist, um die Kopplungsdaten zu lesen.

8. Modul nach Anspruch 7, **dadurch gekennzeichnet, dass** das Ausgangssignal einen Identifikationscode des elektronischen Moduls (20) oder der -Medikamentenabgabevorrichtung (10) darstellt.

9. Modul (20) nach einem der Ansprüche 1 bis 8,
**dadurch gekennzeichnet, dass** die Steuereinrichtung geeignet ist, um eine Verarbeitung von Daten zu beginnen, falls das Vorrichtungseingangssignal aus der Sequenz abgeleitet wird.

10. Modul (20) nach einem der Ansprüche 1 bis 9,
**dadurch gekennzeichnet, dass** die LED-Komponente (21, 22) eine RGB-LED ist.

11. Medikamentenabgabevorrichtung (1) mit einem Behälter zum Aufnehmen eines flüssigen Medikaments und einer Kanüle oder Nadel für eine Abgabe des Medikaments, die Medikamentenabgabevorrichtung (1) umfassend:
eine LED-Komponente (2) mit mindestens zwei Anschlussstiften,
**dadurch gekennzeichnet, dass**
die Medikamentenabgabevorrichtung (1) ferner eine Steuereinrichtung (23), die zum wechselnden Betreiben der LED-Komponente (2) in einem ersten und in einem zweiten Zustand geeignet ist, umfasst,
wobei in dem zweiten Zustand, die Steuereinrichtung eine Spannung über die LED-Komponente (2) hinweg zwischen den mindestens zwei Anschlussstiften überwacht, um eine Sequenz diskreter Änderungen in Lichtintensität, die durch die LED-Komponente (2) detektiert werden, zu bestimmen und um ein Vorrichtungseingangssignal aus der Sequenz abzuleiten, und
wobei in dem ersten Zustand die LED-Komponente (2) durch die Steuereinrichtung (23) betrieben wird, um Lichtimpulse, die ein Ausgangssignal darstellen, zu emittieren.

12. Medikamentenabgabevorrichtung nach Anspruch 11,
**dadurch gekennzeichnet, dass** das Vorrichtungseingangssignal Konfigurationsdaten zum Konfigurieren der Medikamentenabgabevorrichtung darstellt, und wobei die Steuereinrichtung geeignet ist, um die Konfigurationsdaten aus dem Vorrichtungseingangssignal zu lesen.

13. Medikamentenabgabevorrichtung nach Anspruch 12,
**dadurch gekennzeichnet, dass** die Steuereinrichtung konfiguriert ist, um von einem Stromsparmodus in einen Normalbetriebsmodus zu wechseln und um die Verarbeitung von Daten zu beginnen, wenn eine vordefinierte Sequenz diskreter Änderungen in der Intensität absorbierten Lichts detektiert wird.

14. Verfahren zum Einstellen einer Intensität emittierten Lichts einer Anzeige in einer Medikamentenabgabevorrichtung (1), umfassend die Schritte:
a. Betreiben, durch eine Steuereinrichtung (23) der Medikamentenabgabevorrichtung (1), einer LED-Komponente (2) in einem zweiten Zustand;
b. Überwachen, durch die Steuereinrichtung (23), in dem zweiten Zustand eines Spannungsabfalls über die LED-Komponente (2) hinweg zwischen zwei Anschlussstiften der LED-Komponente (2);
c. Ableiten, durch die Steuereinrichtung (23) und basierend auf dem Überwachen, eines Umgebungslichtparameterwerts abhängig von der Eingangslichtintensität, die durch die LED-Komponente (2) detektiert wird;
d. Betreiben, durch die Steuereinrichtung (23), der LED-Komponente (2) in einem ersten Zustand und Emittieren von Licht mit einer Intensität basierend auf dem neuesten Umgebungslichtparameterwert.

15. Verfahren zum Einstellen einer Intensität eines emittierten Lichts in einem elektronischen Modul (20), das an einer Medikamentenabgabevorrichtung (10) lösbar anbringbar ist und zum Überwachen eines Medikamentenabgabeereignisses, das mittels der Medikamentenabgabevorrichtung (10) ausgeführt wird, geeignet ist, das Verfahren umfassend die Schritte:
a. Betreiben, durch eine Steuereinrichtung (23) des elektronischen Moduls (20), einer LED-Komponente (21, 22) in einem zweiten Zustand;
b. Überwachen, durch die Steuereinrichtung (23), in dem zweiten Zustand eines Spannungsabfalls über die LED-Komponente (21, 22) hinweg zwischen zwei Anschlussstiften der LED-Komponente (21, 22);
c. Ableiten, durch die Steuereinrichtung (23) und basierend auf dem Überwachen, eines Umgebungslichtparameterwerts abhängig von der Eingangslichtintensität, die durch die LED-Komponente (21, 22) absorbiert wird;
d. Betreiben, durch die Steuereinrichtung (23), der LED-Komponente (21, 22) in einem ersten Zustand und Emittieren von Licht mit einer Intensität basierend auf dem neuesten Umgebungslichtparameterwert.

## Revendications

1. Module électronique (20) pouvant être fixé de façon libérable à un dispositif de délivrance de médicament (10) et étant conçu pour la surveillance d'un événement de délivrance de médicament exécuté au moyen du dispositif de délivrance de médicament (10), ledit module électronique (20) comprenant
un composant à DEL (21, 22) avec au moins deux broches de connecteur,
**caractérisé en ce que**
le module électronique (20) comprend en outre un organe de commande (23) conçu pour faire fonctionner en alternance le composant à DEL (21, 22) dans un premier et dans un second état,
dans lequel dans le second état, l'organe de commande surveille une tension à travers le composant à DEL (21, 22) entre les au moins deux broches de connecteur, pour déterminer une séquence de changements discrets d'intensité de lumière détectés par le composant à DEL (21, 22), et pour dériver un signal d'entrée de dispositif à partir de la séquence et
dans lequel dans le premier état le composant à DEL (21, 22) est mis en fonctionnement par l'organe de commande (23) pour émettre des impulsions de lumière représentant un signal de sortie.

2. Module (20) selon la revendication 1, **caractérisé par** une unité de capteur permettant de détecter un événement de délivrance de médicament et/ou permettant de détecter des données de dispositif ou des données de médicament d'un médicament étant présent à l'intérieur du dispositif de délivrance de médicament (10), dans lequel l'unité de capteur est conçue pour stocker des données d'événement de délivrance de médicament générées, les données de médicament ou les données de dispositif.

3. Module (20) selon la revendication 2, **caractérisé en ce que** la détection de l'événement de délivrance de médicament comprend la détection d'au moins l'une de ce qui suit ; une quantité de la dernière dose administrée, une indication du moment de la dernière dose administrée, une quantité de la dose suivante à administrer, une indication du moment de la dose suivante à administrer ou la quantité totale de dose administrée.

4. Module (20) selon la revendication 2 ou 3, **caractérisé en ce que** les données de médicament comprennent au moins l'une des informations suivantes ; type de médicament, date d'expiration du médicament ou validité du médicament.

5. Module (20) selon l'une quelconque des revendications 1 à 4, **caractérisé en ce que** l'organe de commande est conçu pour mesurer un taux de changement de la tension à travers le composant à DEL (21, 22).

6. Module (20) selon l'une quelconque des revendications 1 à 5, **caractérisé en ce que** le signal d'entrée de dispositif représente des données de configuration permettant de configurer le module électronique (20) et dans lequel l'organe de commande est conçu pour lire les données de configuration à partir du signal d'entrée de dispositif.

7. Module (20) selon l'une quelconque des revendications 1 à 5, **caractérisé en ce que** le signal d'entrée de dispositif représente des données d'appariement pour l'appariement du module électronique (20) avec un dispositif informatique et dans lequel l'organe de commande est conçu pour lire les données d'appariement.

8. Module selon la revendication 7, **caractérisé en ce que** le signal de sortie représente un code d'identification du module électronique (20) ou du dispositif de délivrance de médicament (10).

9. Module (20) selon l'une quelconque des revendications 1 à 8, **caractérisé en ce que** l'organe de commande est conçu pour démarrer un traitement de données si le signal d'entrée de dispositif est dérivé de la séquence.

10. Module (20) selon l'une quelconque des revendications 1 à 9, **caractérisé en ce que** le composant à DEL (21, 22) est une DEL RVB.

11. Dispositif de délivrance de médicament (1) avec un récipient permettant de contenir un médicament liquide et une canule ou aiguille pour la délivrance du médicament, ledit dispositif de délivrance de médicament (1) comprenant :
un composant à DEL (2) avec au moins deux broches de connecteur,
**caractérisé en ce que**
le dispositif de délivrance de médicament (1) comprend en outre un organe de commande (23) conçu pour faire fonctionner en alternance le composant à DEL (2) dans un premier et dans un second état,
dans lequel dans le second état, l'organe de commande surveille une tension à travers le composant à DEL (2) entre les au moins deux broches de connecteur, pour déterminer une séquence de changements discrets d'intensité de lumière détectés par le composant à DEL (2), et pour dériver un signal d'entrée de dispositif à partir de la séquence et
dans lequel dans le premier état le composant à DEL (2) est mis en fonctionnement par l'organe de commande (23) pour émettre des impulsions de lumière représentant un signal de sortie.

12. Dispositif de délivrance de médicament selon la revendication 11, **caractérisé en ce que** le signal d'entrée de dispositif représente des données de configuration permettant de configurer le dispositif de délivrance de médicament et dans lequel l'organe de commande est conçu pour lire les données de configuration à partir du signal d'entrée de dispositif.

13. Dispositif de délivrance de médicament selon la revendication 12, **caractérisé en ce que** l'organe de commande est configuré pour commuter d'un mode de veille à faible puissance à un mode de fonctionnement normal et pour démarrer un traitement de données lorsqu'une séquence prédéfinie de changements discrets dans l'intensité de lumière absorbée est détectée.

14. Procédé permettant d'ajuster l'intensité de lumière émise d'un affichage dans un dispositif de délivrance de médicament (1) comprenant les étapes consistant à :
a. faire fonctionner, par un organe de commande (23) du dispositif de délivrance de médicament (1), un composant à DEL (2) dans un second état ;
b. surveiller, par l'organe de commande (23), dans le second état un affaiblissement de tension à travers le composant à DEL (2) entre deux broches de connecteur du composant à DEL (2) ;
c. dériver, par l'organe de commande (23) et en fonction de la surveillance, une valeur de paramètre de lumière ambiante dépendant de l'intensité de lumière d'entrée détectée par le composant à DEL (2) ;
d. faire fonctionner, par l'organe de commande (23), le composant à DEL (2) dans un premier état et émettre de la lumière avec une intensité en fonction de la toute dernière valeur de paramètre de lumière ambiante.

15. Procédé permettant d'ajuster l'intensité de lumière émise d'un affichage dans un module électronique (20) pouvant être fixé de façon libérable à un dispositif de délivrance de médicament (10) et étant conçu pour la surveillance d'un événement de délivrance de médicament exécuté au moyen du dispositif de délivrance de médicament (10), le procédé comprenant les étapes consistant à :
a. faire fonctionner, par un organe de commande (23) du module électronique (20), un composant à DEL (21, 22) dans un second état ;
b. surveiller, par l'organe de commande (23), dans le second état un affaiblissement de tension à travers le composant à DEL (21, 22) entre deux broches de connecteur du composant à DEL (21, 22) ;
c. dériver, par l'organe de commande (23) et en fonction de la surveillance, une valeur de paramètre de lumière ambiante dépendant de l'intensité de lumière d'entrée absorbée par le composant à DEL (21, 22) ;
d. faire fonctionner, par l'organe de commande (23), le composant à DEL (21, 22) dans un premier état et émettre de la lumière avec une intensité en fonction de la toute dernière valeur de paramètre de lumière ambiante.
